Europäisches Patentamt

European Patent Office

Office européen des brevets

(11) Numéro de publication: **0 426 556 A1**

# (12) DEMANDE DE BREVET EUROPEEN

(21) Numéro de dépôt: 90403058.2

(22) Date de dépôt: 29.10.90

(51) Int. Cl.⁵: **A61M 5/00**

(30) Priorité: 30.10.89 FR 8914218

(43) Date de publication de la demande:
08.05.91 Bulletin 91/19

(84) Etats contractants désignés:
BE CH DE ES GB IT LI LU NL SE

(71) Demandeur: **Koubbi Humbert, Maurice**
**Blandainville**
**F-28120 Illiers Combray(FR)**

Demandeur: **Raynaud, Henri**
**Chemin du Moulin, Ouilly le Vicomte**
**F-14100 Lisieux(FR)**

(72) Inventeur: **Koubbi Humbert, Maurice**
**Blandainville**
**F-28120 Illiers Combray(FR)**

(74) Mandataire: **Gorree, Jean-Michel et al**
**Cabinet Plasseraud 84, rue d'Amsterdam**
**F-75009 Paris(FR)**

(54) Dispositif de recueil de déchets, et notamment d'aiguilles hypodermiques ou analogues.

(57) Dispositif de recueil utilisable dans le domaine médical pour recevoir des déchets, notamment des aiguilles hypodermiques usagées, agencé sous la forme générale d'un boîtier (1) équipé dans sa partie supérieure d'au moins une ouverture (3) pour l'introduction des déchets et d'un tiroir (4) sous-jacent à cette ouverture et extractible par une paroi du boîtier, et comportant en outre : un bac amovible (5) disposé dans le tiroir (4) pour recueillir ces déchets ; un couvercle amovible (6) emboîtable verticalement sur le bord périphérique du bac un orifice (7) d'introduction du couvercle prévu dans une paroi du boîtier pour l'introduction du couvercle au-dessus du bac avant l'ouverture du tiroir ; et des moyens de guidage solidaires du boîtier et situés à l'intérieur de celui-ci et à hauteur approximative du bord supérieur du bac, entre la paroi avant du boîtier et le bac, ces moyens de guidage comprenant au moins une rampe (10) inclinée du haut vers le bas d'arrière en avant de manière que, lors de l'ouverture du tiroir, le couvercle, simplement posé sur le bac, vienne en contact avec la rampe et que celle-ci engendre sur le couvercle une composante de force verticale dirigée vers le bas qui provoque l'emboîtement du couvercle sur le bac, ce grâce à quoi, une fois le tiroir entièrement ouvert, le bac apparait fermé par son couvercle.

FIG.1

## DISPOSITIF DE RECUEIL DE DÉCHETS, ET NOTAMMENT D'AIGUILLES HYPODERMIQUES OU ANALOGUES

La présente invention concerne des perfectionnements apportés aux dispositifs de recueil utilisables dans le domaine médical, et notamment en milieu hospitalier ou analogue, pour recevoir des déchets pollués et/ou dangereux et agencés pour que les personnes l'utilisant ou le manipulant ne puissent pas toucher par inadvertance lesdits déchets, ce dispositif étant agencé sous la forme générale d'un boîtier équipé dans sa partie supérieure d'au moins une ouverture pour l'introduction des déchets et d'un tiroir sous-jacent à cette ouverture et extractible par une paroi (paroi avant) du boîtier.

L'invention concerne en particulier, mais non exclusivement, des dispositifs du genre ci-dessus destinés à recueillir des aiguilles hypodermiques usagées, des seringues usagées équipées d'aiguilles hypodermiques, ou d'autres objets analogues du type pointu et/ou acéré.

Il est souhaitable, dans un but d'hygiène et de sécurité, que les déchets contaminés résultant des soins apportés à des patients soient recueillis dans des récipients dans lesquels ils ne puissent pas être touchés par inadvertance, afin d'empêcher les risques de propagation des maladies, les risques d'infection ou les risques d'accident.

En particulier les aiguilles hypodermiques ou autres objets analogues pointus ou acérés peuvent, après utilisation, conserver des traces de sang contaminé ou de tissus contaminés d'un patient, ou simplement véhiculer des microbes, virus, etc. Ces aiguilles à jeter font donc courir le risque qu'une personne puisse se blesser sur la pointe (piqûre, coupure, déchirure) et/ou également le risque qu'une personne puisse être contaminée en touchant cette pointe ou a fortiori en se blessant.

Dans la demande de brevet FR. 2 625 904, on a déjà proposé un dispositif permettant de recueillir les aiguilles hypodermiques usagées, dispositif qui était agencé de manière que l'aiguille puisse être séparée mécaniquement de la seringue en n'utilisant qu'une seule main tenant la seringue exclusivement (donc sans qu'il y ait contact de l'aiguille avec l'autre main) et que cette aiguille, une fois détachée, tombe par gravité dans un tiroir de recueil.

Bien que ce dispositif donne entière satisfaction quant aux fonctions spécifiques pour lesquelles il a été prévu, il s'avère cependant nécessaire d'accroître encore la sécurité et de faire en sorte que les utilisateurs d'un dispositif de recueil de déchets de ce genre non seulement ne puissent pas toucher les déchets au cours de la phase d'utilisation des déchets, mais également ne puissent pas les toucher lors de l'évacuation de ces déchets hors du dispositif (extraction du tiroir).

C'est donc le but de la présente invention que de proposer un agencement perfectionné d'un dispositif du genre précité destiné au recueil de déchets et notamment d'objets pointus ou acérés, tels que des aiguilles hypodermiques usagées, qui évite tout risque de contact accidentel pendant de la phase de remplissage du dispositif et ultérieurement.

A cette fin, l'invention propose un dispositif de recueil utilisable dans le domaine médical, et notamment en milieu hospotalier ou analogue, pour recevoir des déchets pollués et/ou dangereux et agencé pour que les personnes l'utilisant ou le manipulant ne puissent pas toucher lesdits déchets, ce dispositif étant agencé sous la forme générale d'un boîtier équipé dans sa partie supérieure d'au moins une ouverture pour l'introduction des déchets et d'un tiroir sous-jacent à cette ouverture et extractible par une paroi (paroi avant) du boîtier,
caractérisé en ce qu'il comporte en outre :
- un bac amovible disposé dans le tiroir et destiné à recueillir lesdits déchets, ce bac étant ouvert à sa partie supérieure,
- un couvercle amovible pour le bac et emboîtable verticalement sur le bord périphérique de celui-ci,
- un orifice d'introduction du couvercle prévu dans une paroi du boîtier pour l'introduction du couvercle au-dessus du bac avant l'ouverture du tiroir,
- et des moyens de guidage solidaires du boîtier et situés à l'intérieur de celui-ci et à hauteur approximative du bord supérieur du bac, entre la paroi avant du boîtier et le bac, ces moyens de guidage comprenant au moins une rampe inclinée du haut vers le bas d'arrière en avant (sens de déplacement du tiroir lors de l'ouverture) de manière que, lors de l'ouverture du tiroir, le couvercle, préalablement introduit par l'orifice de mise en place et simplement posé sur le bac, vienne en contact avec la rampe et que celle-ci engendre sur le couvercle une composante de force verticale dirigée vers le bas qui provoque l'emboîtement du couvercle sur le bac, ce grâce à quoi, une fois le tiroir entièrement ouvert, le bac apparait fermé par son couvercle.

En visant une application particulière, l'invention propose également un dispositif de recueil utilisable notamment en milieu hospitalier ou analogue, pour recevoir des aiguilles hypodermiques usagées à jeter, des seringes usagées à jeter équipées d'aiguilles hypodermiques, ou d'autres objets analogues, agencé pour que les personnes l'utilisant ou le manipulant ne puissent pas toucher lesdites aiguilles ou analogues, ce dispositif étant agencé sous la forme générale d'un boîtier équipé dans sa partie supérieure d'au moins une ouverture pour l'introduction desdites aiguilles ou analogues et d'un tiroir sous-jacent à cette ouverture et extractible par une paroi (paroi avant) du boîtier, caractérisé en ce qu'il comporte en outre :
- un bac amovible disposé dans le tiroir et destiné à recueillir lesdites aiguilles, ce bac étant ouvert à sa partie supérieure,
- un couvercle amovible pour le bac et emboîtable verticalement sur le bord périphérique de celui-ci,
- un orifice d'introduction du couvercle prévu dans une paroi du boîtier pour l'introduction du couvercle au-dessus du bac avant l'ouverture du tiroir,
- et des moyens de guidage solidaires du boîtier et situés à l'intérieur de celui-ci et à hauteur approximative du bord supérieur du bac, entre la paroi avant du boîtier et le bac, ces moyens de guidage comprenant au moins une rampe inclinée du haut vers le bas d'arrière en avant (sens de déplacement du tiroir lors de l'ouverture) de manière que, lors de l'ouverture du tiroir, le couvercle, préalablement introduit par l'orifice de mise en place et simplement posé sur le bac, vienne en contact avec la rampe et que celle-ci engendre sur le couvercle une composante de force verticale dirigée vers le bas qui provoque l'emboîtement du couvercle sur le bac, ce grâce à quoi, une fois le tiroir entièrement ouvert, le bac apparait fermé par son couvercle.

Ainsi grâce aux dispositions conformes à l'invention, les déchets introduits dans le dispositif et recueillis dans le bac ne peuvent pas être touchés par inadvertence lorsque le bac est enfermé dans le boîtier (les jeux des organes mobiles et les dimensions des diverses ouvertures -orifice d'introduction des déchets, orifice d'introduction du couvercle- peuvent être suffisamment faibles pour interdire le passage involontaire des doigts). En outre, lors de l'ouverture du tiroir, le bac apparaît fermé et là encore il n'y a aucun risque de contact involontaire. La sécurité est donc considérablement accrue.

Pour assurer que le couvercle, au moment de son introduction, se mette correctement en place sur le dessus du bac, il est souhaitable que le bac se trouve dans une position parfaitement déterminée dans le tiroir qui, notamment en raison de la présence des moyens de guidage, doit posséder des dimensions (notamment longitudinales) supérieures à celles du bac : à cette fin, on prévoit que le tiroir soit équipé de moyens de positionnement agencés pour maintenir le bac dans une position prédéterminée par rapport aux parois, et notamment à la paroi avant, du tiroir. Toujours dans le même but d'une mise en place correcte du couvercle sur le bac, le dispositif peut en outre comporter avantageusement des moyens de butée agencés pour arrêter le déplacement du couvercle, lors de son introduction, lorsque celui-ci parvient approximativement au-dessus de la susdite position prédéterminée du bac.

Dans un mode de réalisation préféré qui permet d'obtenir un emboîtement sûr du couvercle sans que soient engendrées des forces de frottement trop importantes, et donc sans qu'il soit nécessaire de développer un effort trop important pour extraire le tiroir hors du boîtier, il est prévu que la rampe de guidage soit discontinue et comprenne au moins deux tronçons latéraux situés respectivement au droit des parois latérales du bac et aptes à coagir avec les deux bords latéraux respectifs du couvercle sur toute la longueur desdits bords latéraux.

Toutefois, dans ce cas, il est souhaitable que la rampe de guidage comprenne en outre un tronçon approximativement central apte à coagir avec une portion centrale des bords antérieur et postérieur du couvercle, de manière à assurer un emboîtement complet du couvercle sur tous ses côtés.

De façon avantageuse, sur la face externe de la paroi du boîtier munie de l'orifice d'introduction du couvercle, sont prévus des moyens de support qui sont agencés de manière à recevoir le couvercle en position verticale lorsque le dispositif est en cours d'utilisation et qui sont positionnés de manière que le couvercle ainsi supporté s'étende devant l'orifice d'introduction et empêche l'accès, a travers celui-ci, à l'intérieur du boîtier. Le couvercle reste ainsi associé physiquement au boiter au cours de la phase de remplissage du bac et ne risque pas d'être égaré. En outre, dans sa position de rangement, le couvercle s'étend devant l'orifice d'introduction prévu pour sa mise en place et empêche l'accès, par cette voie, à l'intérieur du bac. La sécurité s'en trouve encore accrue.

La facilité de conception et d'utilisation du dispositif semble être optimale lorsque la paroi du boîtier munie de l'orifice d'introduction du couvercle est la paroi arrière.

Le fonctionnement convenable du dispositif nécessite que le couvercle puisse être solidarisé au bac de façon simple, mais efficace, et qu'il ne puisse pas ensuite s'en détacher de façon intempestive. Il est donc souhaitable que les moyens d'accrochage mutuel du couvercle et du bac soient appropriés en conséquence et, à cette fin, on prévoit de façon avantageuse que le couvercle possède une gorge périphérique ouverte vers le dessous du couvercle et présentant au moins une lèvre interne continue d'accrochage et en ce que le bac possède au moins un épaulement externe suivant continuement le bord libre périphérique de son ouverture et agencé pour coopérer avec la susdite lèvre du couvercle.

Toujours pour accroître la sécurité et renforcer la solidarisation du couvercle au bac, on peut en outre prévoir des pattes discontinues de verrouillage disposées sur le couvercle ou le bac pour coopérer, en position de fermeture du bac, avec des épaulements de retenue prévus respectivement sur le bac ou le couvercle pour verrouiller le couvercle en position de fermeture sur le bac.

Dans le cas spécifique du recueil d'aiguilles hypodermiques et analogues, il convient d'éviter que ces objets pointus ou acérés puissent perforer le bac fermé par le couvercle. Il est donc nécessaire que le bac et le couvercle soient constitués en des matériaux respectifs, notamment en polystyrène, non susceptibles d'être perforés par les aiguilles ou analogues.

Enfin, il est souhaitable que les objets recueillis puissent être détruits sans qu'il soit nécessaire à aucun moment d'ouvrir le bac dans lequel ils ont été recueillis. De ce fait, il est souhaitable que les matériaux constitutifs respectivement du bac et du couvercle soient en outre incinérable.

L'invention sera mieux comprise à la lecture de la description détaillée qui suit d'un mode de réalisation préféré, donné uniquement à titre d'exemple non limitatif. Dans cette description, on se réfère aux dessins annexés sur lesquels :

- la figure 1 est une vue schématique en perspective, de dessus et de devant, d'un dispositif de recueil de déchets agencé conformément à l'invention ;
- la figure 2 est une vue schématique partielle, également en perspective, montrant l'arrière du dispositif de la figure 1 ; et
- la figure 3 est une vue schématique de côté en coupe du dispositif de la figure 1.

Parce que c'est dans cette application que le dispositif de l'invention semble devoir procurer les avantages les plus importants, le dispositif qui va être décrit et qui est représenté aux figures est destiné plus spécifiquement à recueillir des aiguille hypodermiques usagées. A cet effet, le dispositif comporte un boîtier 1 dont la face supérieure comporte, selon une disposition de la demande de brevet FR. 2 625 904, un tableau 2 muni d'ouvertures 3 configurées pour autoriser le démontage des aiguilles hypodermiques en tenant d'une seule main la seringue qui les supporte.

Mais on comprendra, d'après la suite de la description, que les dispositions propres à l'invention ne sont pas limitées à cette seule application et que le boîtier peut être agencé différemment pour recevoir d'autres types de déchets (tampons, pansements, etc.) en mettant en oeuvre les dispositions de l'invention.

A l'intérieur du boîtier 1 est disposé un tiroir 4 apte à être extrait par la face avant (évidée) du boîtier. Dans le tiroir 4 est disposé un bac amovible 5 ouvert à sa partie supérieure et constitué (de même que son couvercle dont il sera question plus loin) en un matériau résistant aux perforations (par exemple du polystyrène) et incinérable.

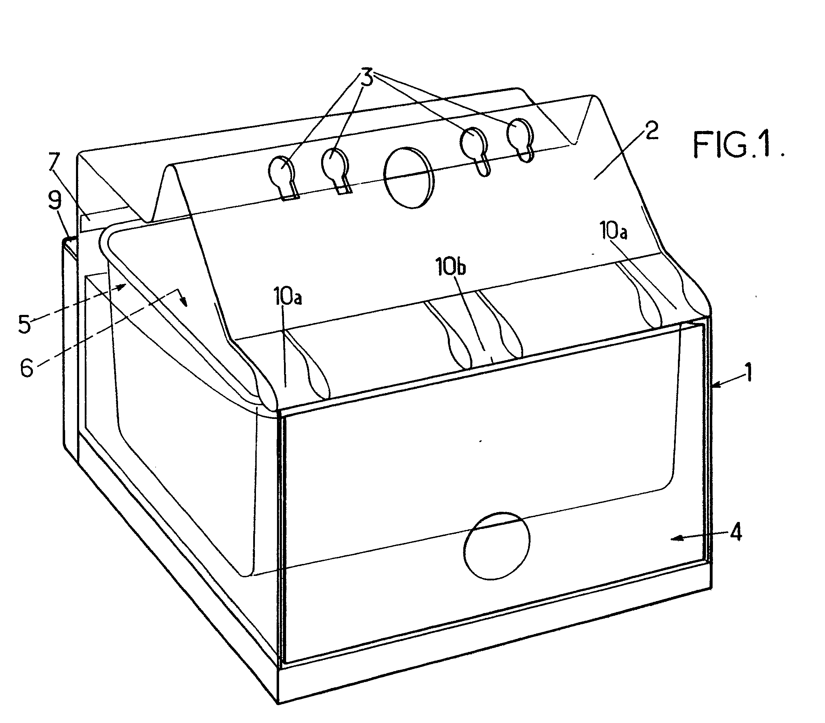

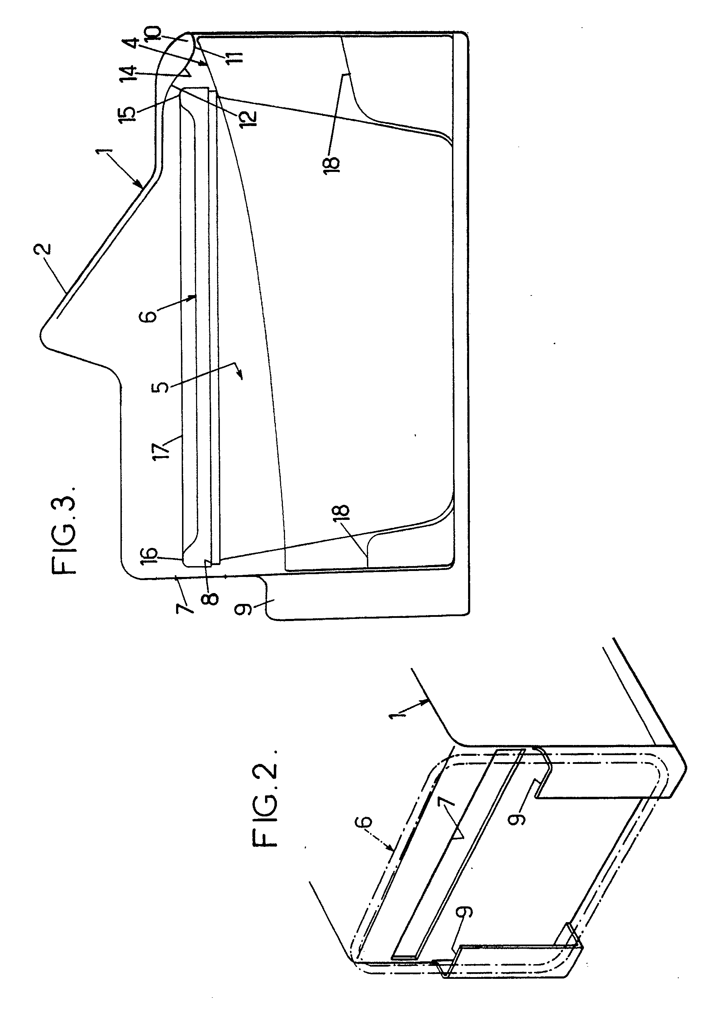

Dans la position représentée aux figure 1 et 3, et conformément au mode de fonctionnement décrit dans la demande de brevet FR. 2 625 904, les aiguilles introduites par les ouvertures 3 tombent par gravité dans le bac 5.

Le bac 5 est obturable à l'aide d'un couvercle 6 qui est emboîtable sur le bord périphérique libre de l'ouverture du bac, lorsqu'on exerce sur lui un effort vertical. Les moyens d'accrochage du couvercle sur le bac peuvent être de types utilisés couramment, par exemple pour des récipients à usage alimentaire : de façon schématique, le couvercle possède alors une gorge périphérique ouverte vers le dessous du couvercle et présentant au moins une lèvre interne continue d'accrochage et le bac comporte au moins un épaulement externe suivant continuement le bord périphérique de son ouverture et agencé pour coopérer avec la lèvre du couvercle. Bien entendu de nombreuses variantes sont possibles.

De plus, ou en substitution, on peut prévoir des pattes de verrouillage prévues sur le couvercle ou de préférence sur le bac, pour coopérer avec des épaulements de retenue prévus respectivement sur le bac ou le couvercle pour verrouiller le couvercle en position de fermeture sur le bac.

Pour la mise en place du couvercle sur le bac, on prévoit un orifice allongé d'introduction 7 percé dans la paroi arrière du boîtier, orifice qui est situé à hauteur ou légèrement au-dessus du niveau du bord supérieur 8 du bac 5 disposé dans le tiroir 4 et qui s'étend sensiblement sur toute la largeur de cette paroi arrière.

Accessoirement, on peut prévoir que la paroi arrière soit munie extérieurement de deux gouttières verticales 9 formant glissières, dans lesquelles le couvercle 6 peut être introduit en position verticale. Lors de la phase de remplissage du bac, on évite ainsi d'égarer le couvercle qui reste physiquement associé au boîtier, et en outre le couvercle ainsi disposé (voir fig. 2) cache l'orifice 7 et empêche d'y passer les doigts et d'accéder au contenu du bac.

Pour que, conformément au but poursuivi à des fins de sécurité, le bac apparaisse fermé avec le couvercle emboîté sur lui lorsque le tiroir 4 est extrait du boîtier 1, on prévoit des moyens d'emboîtement du couvercle sur le bac qui sont agencés comme suit. A l'intérieur du boîtier, et à la partie antérieure supérieure de celui-ci, est disposée une rampe 10 inclinée de haut en bas de l'arrière vers l'avant (en considérant le sens de sortie du tiroir). Cette rampe 10 possède un bord inférieur 11 (situé vers l'avant) qui est disposé à une hauteur correspondant approximativement au bord supérieur du bac, ou plus précisément à la position verticale de la face supérieure 15 du couvercle 6 complètement emboîté sur le bord du bac.

La rampe 10 peut avoir une inclinaison quelconque apte à engendrer sur le bord du couvercle 6 glissant sur elle une composante de force verticale dirigée vers le bas et suffisante pour provoquer l'enfoncement du couvercle et son emboîtement sur le bac ; il faut cependant faire en sorte que la composante de force dirigée horizontalement, et qui s'oppose à la sortie du tiroir, ne soit pas d'une importance exagérée et n'oblige pas à exercer sur le tiroir un effort trop important. Une inclinaison d'environ 20 à 45° sur l'horizontale semble convenir.

De plus, on pourra donner à la rampe une forme curviligne avec une inclinaison moindre dans ses parties extrêmement 12, 13 et une inclinaison plus accentuée dans sa partie centrale 14 comme visible à la fig. 3. On diminue alors l'importance de l'effort résistant engendré au début du déplacement du tiroir lorsque la rampe frotte sur toute la longueur des bords antérieur 15 et postérieur 16 du couvercle et provoque le mouvement vers le bas desdits bords antérieur et postérieur du couvercle pour leur emboîtement sur les bords antérieur et postérieur du bac.

Egalement, dans le but de diminuer les efforts au moment de l'emboîtement des bords antérieur et postérieur du couvercle, on prévoit que la rampe 10 n'est pas constituée sous forme unitaire, mais s'étend de façon discontinue sur la largeur du boîtier : ainsi deux tronçons latéraux 10a sont situés sur la trajectoire suivie par les bords longitudinaux 17 du couvercle et provoquent leur emboîtement sur toute leur longueur et un tronçon central 10b n'intervient que pour parfaire l'emboîtement des bords antérieur 15 et postérieur 16 pour lesquels l'action des seuls tronçons latéraux 10a risquerait d'être insuffisante.

On notera que, dans la position montrée à la fig. 3, le couvercle 6 est simplement posé sur le bac 5, mais n'est pas emboîté.

Comme cela est visible à la fig. 3, la présence de la rampe 10 oblige à laisser un intervalle entre les faces avant du bac et du tiroir. En outre, pour que la mise en place du bac dans le tiroir et son enlèvement hors de celui-ci puissent s'effectuer facilement, il est préférable que les dimensions horizontales du bac soient sensiblement inférieures à celles du tiroir ; en outre, le bac peut présenter une forme pyramidale alors que le tiroir est de forme parallélépipédique (cas représenté). Or, il est nécessaire que le bac occupe une position bien définie par rapport aux parois du tiroir et par rapport à l'orifice d'introduction 7 de manière que le couvercle, lors de son introduction, se positionne de façon certaine sur le bord périphérique de l'ouverture du bac ; il faut, d'autre part, empêcher le bac de basculer au moment de l'ouverture du tiroir, sous l'action des efforts verticaux engendrés par la rampe. On prévoit donc des moyens de positionnement du bac dans le tiroir, moyens constitués par exemple par des bossages 18 discontinus ou un bossage périphérique continu saillants sur les faces internes du tiroir, notamment à sa partie inférieure.

Enfin, des moyens de butée doivent être prévus pour limiter la course d'introduction du couvercle et faire en sorte qu'il retombe exactement sur le bac. Ces moyens de butées peuvent avantageusement être constitués par la rampe 10 elle-même, dont la partie postérieure 12 peut s'étendre jusqu'au droit du bord antérieur du bac.

Comme il va de soi et comme il résulte d'ailleurs déjà de ce qui précède, l'invention ne se limite nullement à ceux de ses modes d'application et de réalisation qui ont été plus particulièrement envisagés ; elle en embrasse, au contraire, toutes les variantes.

## Revendications

1. Dispositif de recueil utilisable dans le domaine médical, et notamment en milieu hospitalier ou analogue, pour recevoir des déchets pollués et/ou dangereux et agencé pour que les personnes l'utilisant ou le manipulant ne puissent pas toucher lesdits déchets, ce dispositif étant agencé sous la forme générale d'un boîtier (1) équipé dans sa partie supérieure d'au moins une ouverture (3) pour l'introduction des déchets et d'un tiroir (4) sous-jacent à cette ouverture et extractible par une paroi (paroi avant) du boîtier,
caractérisé en ce qu'il comporte en outre :
- un bac amovible (5) disposé dans le tiroir (4) et destiné à recueillir lesdits déchets, ce bac étant ouvert à sa partie supérieure,
- un couvercle amovible (6) pour le bac et emboîtable verticalement sur le bord périphérique de celui-ci,
- un orifice (7) d'introduction du couvercle prévu dans une paroi du boîtier pour l'introduction du couvercle au-dessus du bac avant l'ouverture du tiroir,
- et des moyens de guidage solidaires du boîtier et situés à l'intérieur de celui-ci et à hauteur approximative du bord supérieur du bac, entre la paroi avant du boîtier et le bac, ces moyens de guidage comprenant au moins une rampe (10) inclinée du haut vers le bas d'arrière en avant (sens de déplacement du tiroir lors de l'ouverture) de manière que, lors de l'ouverture du tiroir, le couvercle, préalablement introduit par l'orifice de mise en place et simplement posé sur le bac, vienne en contact avec la rampe et que celle-ci engendre sur le couvercle une composante de force verticale dirigée vers le bas qui provoque l'emboîtement du couvercle sur le bac, ce grâce a quoi, une fois le tiroir entièrement ouvert, le bac apparait fermé par son couvercle.

2. Dispositif de recueil utilisable notamment en milieu hospitalier ou analogue, pour recevoir des aiguilles hypodermiques usagées à jeter, des seringues équipées d'aiguilles hypodermiques usagées à jeter, ou d'autres objets analogues, agencé pour que les personnes l'utilisant ou le manipulant ne puissent pas toucher lesdits déchets, ce dispositif étant agencé sous la forme générale d'un boîtier (1) équipé dans sa partie supérieure d'au moins une ouverture (3) pour l'introduction desdites aiguilles ou analogues et d'un tiroir (4) sous-jacent à cette ouverture et extractible par une paroi (paroi avant) du boîtier,

caractérisé en ce qu'il comporte en outre :

- un bac amovible (5) disposé dans le tiroir (4) et destiné à recueillir lesdites aiguilles, ce bac étant ouvert à sa partie supérieure,

- un couvercle amovible (6) pour le bac et emboîtable verticalement sur le bord périphérique de celui-ci,

- un orifice d'introduction du couvercle prévu dans une paroi du boîtier pour l'introduction du couvercle au-dessus du bac avant l'ouverture du tiroir,

- et des moyens de guidage solidaires du boîtier et situés à l'intérieur de celui-ci et à hauteur approximative du bord supérieur du bac, entre la paroi avant du boîtier et le bac, ces moyens de guidage comprenant au moins une rampe (10) inclinée du haut vers le bas d'arrière en avant (sens de déplacement du tiroir lors de l'ouverture) de manière que, lors de l'ouverture du tiroir, le couvercle, préalablement introduit par l'orifice de mise en place et simplement posé sur le bac, vienne en contact avec la rampe et que celle-ci engendre sur le couvercle une composante de force verticale dirigée vers le bas qui provoque l'emboîtement du couvercle sur le bac, ce grâce à quoi, une fois le tiroir entièrement ouvert, le bac apparait fermé par son couvercle.

3. Dispositif de recueil selon la revendication 1 ou 2, caractérisé en ce que le tiroir est équipé de moyens de positionnement (18) agencés pour maintenir le bac dans une position prédéterminée par rapport aux parois, et notamment à la paroi avant, du tiroir.

4. Dispositif de recueil selon la revendication 3, caractérisé en ce qu'il comporte en outre des moyens de butée (12) agencés pour arrêter le déplacement du couvercle, lors de son introduction, lorsque celui-ci parvient approximativement au-dessus de la susdite position prédéterminée du bac.

5. Dispositif de recueil selon l'une quelconque des revendications 1 à 4, caractérisé en ce que la rampe (10) de guidage est discontinue et comprend au moins deux tronçons (10a ) latéraux situés respectivement au droit des parois latérales du bac et aptes à coagir avec les deux bords latéraux respectifs du couvercle sur toute la longueur desdits bords latéraux.

6. Dispositif de recueil selon la revendication 5, caractérisé en ce que la rampe de guidage comprend en outre un tronçon approximativement central (10b) apte à coagir avec une portion centrale des bords antérieur (15) et postérieur (16) du couvercle.

7. Dispositif de recueil selon l'une quelconque des revendications 1 à 6, caractérisé en ce que, sur la face externe de la paroi du boîtier munie de l'orifice d'introduction du couvercle, sont prévus des moyens de support (9) qui sont agencés de manière à recevoir le couvercle (6) en position verticale lorsque le dispositif est en cours d'utilisation et qui sont positionnés de manière que le couvercle ainsi supporté s'étende devant l'orifice d'introduction (7) et empêche l'accès, à travers celui-ci, à l'intérieur du boîtier.

8. Dispositif de recueil selon l'une quelconque des revendications 1 à 7, caractérisé en ce que la paroi du boîtier munie de l'orifice d'introduction (7) du couvercle est la paroi arrière.

9. Dispositif de recueil selon l'une quelconque des revendications 1 à 8, caractérisé en ce que le couvercle possède une gorge périphérique ouverte vers le dessous du couvercle et présentant au moins une lèvre interne continue d'accrochage et en ce que le bac possède au moins un épaulement externe suivant continuement le bord libre périphérique de son ouverture et, agencé pour coopérer avec la susdite lèvre du couvercle.

10. Dispositif de recueil selon l'une quelconque des revendications 1 à 8, caractérisé en ce qu'il est en outre prévu des pattes discontinues dè verrouillage prévues sur le couvercle ou le bac pour coopérer en position de fermeture du bac avec des épaulements de retenue prévus respectivement sur le bac ou le couvercle pour verrouiller le couvercle en position de fermeture sur le bac.

11. Dispositif de recueil selon l'une quelconque des revendications 2 à 10, caractérisé en ce que le bac et le couvercle sont constitués en des matériaux respectifs, notamment en polystyrène, non susceptibles d'être perforés par les aiguilles.

12. Dispositif de recueil selon la revendication 11, caractérisé en ce que les matériaux respectifs constitutifs du bac et du couvercle sont incinérables.

FIG.1.

FIG.2.

FIG.3.

EP 0 426 556 A1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A,D | FR-A-2 625 904 (KOUBBI-HUMBERT)<br>* Figure 1 * | 1 | A 61 M 5/00 |
| A | US-A-3 408 789 (J.A. REDDICK)<br>* Figure 3; colonne 3, lignes 38-50 * | 1 | |
| A | US-A-4 848 570 (GOSCINIAK)<br>* Figure 1; abrégé * | 1 | |

DOMAINES TECHNIQUES RECHERCHES (Int. Cl.5)

A 61 M
A 61 B

Le présent rapport de recherche a été établi pour toutes les revendications

| Lieu de la recherche | Date d'achèvement de la recherche | Examinateur |
|---|---|---|
| La Haye | 05 février 91 | SEDY, R. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention

E : document de brevet antérieur, mais publié à la date de dépôt ou après cette date
D : cité dans la demande
L : cité pour d'autres raisons

 

& : membre de la même famille, document correspondant